# EUROPEAN PATENT SPECIFICATION

(11) **EP 4 346 700 B1**
(45) Date of publication and mention of the grant of the patent: **11.02.2026**
(21) Application number: 22736013.8
(22) Date of filing: 24.05.2022
(51) Int. Cl.: A61F 2/24

(54) **SELF-EXPANDABLE PROSTHETIC HEART VALVE**
SELBSTEXPANDIERBARE HERZKLAPPENPROTHESE
VALVULE CARDIAQUE PROTHÉTIQUE AUTO-EXTENSIBLE

(30) Priority: 25.05.2021 IT 202100013559
(43) Date of publication of application: 10.04.2024
(73) Proprietor: Tarantini, Giuseppe, 35128 Padova (IT)
(72) Inventor: Tarantini, Giuseppe, 35128 Padova (IT)
(74) Representative: Petraz, Gilberto Luigi
(86) International application number: PCT/IT2022/050144
(87) International publication number: WO 2022/249211

(56) References cited:
- EP-A1- 2 537 487
- US-A1- 2011 264 191
- US-A1- 2020 146 824
- US-A1- 2020 330 251

## Description

### FIELD OF THE INVENTION

The present invention concerns a prosthetic heart valve of the self-expandable type for the percutaneous correction of heart valve defects, in particular for the replacement of a native aortic valve. In particular, the prosthetic heart valve can be advantageously used for the correction of pure aortic insufficiency.

### BACKGROUND OF THE INVENTION

It is known that the percutaneous (that is, transcatheter) treatment of patients with symptomatic pure aortic insufficiency (that is, without associated stenosis), and who are considered inoperable with traditional surgery or at high surgical risk, represents an important "unmet need" in current clinical practice.

Aortic prostheses implanted percutaneously are, in fact, designed for the treatment or correction of stenotic aortic valve disease, that is, the presence of a narrowing of the valve orifice resulting mostly from a fibrocalcific degenerative process that affects the aortic cusps, limiting their opening during cardiac systole.

There are two main types of transcatheter prostheses:
1) "self-expandable" prostheses, which are self-expandable thanks to a particular physical property of the metal alloy (usually nitinol) of which they are made,
2) "balloon-expandable" prostheses, that is, attached to a balloon which is then inflated at the level of the aortic valve, causing the expansion of the prosthesis itself.

The implantation of each prosthesis requires the presence of an adequate quantity of calcium at the valve level in order to guarantee the stable anchoring of the prosthesis.

One disadvantage deriving from these anatomical requirements is the problematic implantation of a transcatheter aortic prosthesis at the level of an insufficient aortic valve, in which the calcifications mentioned above are absent or present only in small quantities.

In addition, aortic valve insufficiency is frequently associated with the dilation of the aortic root, that is, the portion of the aorta at the level of which the aortic cusps are naturally inserted and which, if dilated, can lead to a coaptation deficit of the valve leaflets.

Consequently, another disadvantage is that the aortic valve ring, defined by the most caudal portion (nadir) of the three aortic cusps (the so-called anatomical "virtual basal ring"), and at the level of which the prosthesis is usually implanted percutaneously, may exceed the maximum sizes allowed by the size charts of the prostheses on the market.

To date, in very selected cases it is possible to implant a prosthesis percutaneously in order to treat pure aortic insufficiency with an off-label use, that is, a use outside of the proven scope and approval marks, overcoming the problem of the lack or limited presence of valve calcium by oversizing the transcatheter prostheses, thus ensuring a stable anchorage.

Another disadvantage is that, in this anatomical context, an anchoring mechanism based almost exclusively on prosthetic oversizing can be associated with a greater risk of embolization/migration of the implanted prosthesis.

Another disadvantage is that, due to the dilation of the aortic root, the sizes of the aortic ring may be such as not to guarantee adequate oversizing of the transcatheter prosthesis, making this option impractical.

Transcatheter prostheses are also known whose indications also include the treatment of pure aortic insufficiency. The technology behind the release and implantation of this known prosthesis consists of a mechanism that is divided into two main steps, of which the first fundamental one is represented by the opening of three arms ("locators") which are then directed and positioned at the base of the three sinuses of Valsalva (structures delimited by the insertion of the aortic cusps), and which act as a "clip" by means of which the body of the prosthesis, released in the second step, is anchored to the aortic valve.

However, this system of release and anchoring of the prosthesis has some technical disadvantages:
1) the opening of the locators is a one-way step, meaning that it is not possible to recapture these mechanical elements inside the introducer that accommodates the prosthesis, and
2) the second step is also not reversible, which is why, in the event of incorrect positioning or choice of size, the risk of embolization/migration of the prosthesis is high, with no possibility of being able to recapture the prosthesis to then reposition it.

Document US 2011/264191 A1 discloses delivery systems and methods of implantation for prosthetic heart valves. Document EP 2537487 A1 discloses a repositionable heart valve. Document US 2020/146824 A1 discloses delivery of a prosthetic valve. Document US 2020/330251 A1 discloses a connection release structure and system thereof.

There is therefore the need to perfect a prosthetic heart valve that can overcome at least one of the disadvantages of the state of the art.

In particular, one purpose of the present invention is to provide a prosthetic heart valve that allows the defects affecting the native aortic valve, in particular in pure aortic insufficiency, to be corrected percutaneously, avoiding the disadvantages of the state of the art in relation to the difficulty in correctly and safely implanting a prosthetic heart valve.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the present invention or variants to the main inventive idea.

A prosthetic heart valve of the self-expandable type according to the present invention, for the percutaneous correction of heart valve defects, in particular for the replacement of a native aortic valve, is according to the appended claims and suitable to be inserted into a catheter in order to be introduced into a body, by a medical operator, by means of an insertion tool which can be associated with the catheter.

The prosthetic heart valve comprises a support structure configured to self-expand, in a substantially radial manner, from a first compressed conformation, when inserted into the catheter, to a second expanded, or implant, conformation, when removed from the catheter, and a valve prosthesis, configured to replace the native aortic valve, disposed inside the support structure.

The support structure is made of a shape-memory material, such as a metal alloy, in particular nitinol.

The passage of the support structure from the first compressed conformation to the second expanded conformation can occur gradually as it is removed from the catheter or from another possible containing casing in which it is inserted.

According to one aspect of the present invention, the prosthetic heart valve also comprises connection means which can be inserted into the catheter, and which can be connected, at a first end, or proximal end, to the insertion tool and, at a second opposite end, or distal end, to the support structure in a removable manner, wherein the connection means are configured to allow the selective and reversible control, by means of the insertion tool, of the conformation of the support structure.

In this way, the surgeon is able to check the conformation of the support structure during the positioning operations of the prosthetic heart valve with respect to the native aortic valve. Consequently, the release of the support structure is commanded only when the surgeon judges its positioning to be correct and safe. On the contrary, in the event of conditions which prevent its correct and safe attachment, for example an unfavorable anatomical context, the connection means allow to recover the valve prosthesis and avoid serious consequences for the patient.

The support structure comprises an upper portion provided with attachment means, which are configured to be attached to the cusps of the native aortic valve, with which the connection means and a lower portion that contains the valve prosthesis can be associated. The attachment means comprises a ring-type structure which extroflects from said upper portion or a plurality of elongated arms, independent of each other, one for each cusp of the native aortic valve.

According to another aspect of the invention, the upper portion comprises a plurality of connectors configured to be associated with the connection means.

According to another aspect of the invention, the connection means are provided with coupling means configured to connect to each of the connectors in a removable manner.

According to another aspect of the invention, the coupling means comprise two arms hinged at the end of the respective connection means in order to rotate about an axis of rotation so as to be associated with the connectors and, moreover, their rotation can be selectively controlled by the insertion tool.

According to another aspect of the invention, the ring-type structure is made of nitinol fibers intertwined to form a mesh, or grid, suitable to filter the debris released during implant operations.

In particular, the mesh has a weft with a gap of such sizes as to allow the anterograde blood flow even after the complete expansion of the upper portion (and therefore before the complete opening of the lower portion in which the prosthetic valve apparatus is located).

According to another aspect of the invention, the lower portion and the attachment means comprise a plurality of magnetic elements, so that in the expanded conformation as above the plurality of magnetic elements of the lower portion and of the attachment means are sufficiently close for a magnetic attraction force to form between them.

The prosthetic heart valve is inserted into a capsule which comprises a proximal portion and a distal portion connected to each other in a separable manner, the upper portion being contained in the proximal portion and the lower portion being contained in the distal portion.

In this way, it is possible to remove, in an independent manner, one or the other of either the lower portion or the upper portion of the support structure. It follows that it is possible to selectively control which one of the two portions, lower or upper, has to be the first to assume the expanded conformation, while maintaining the other in the compressed form in the desired manner.

Advantageously, this allows the surgeon to obtain a fine control of the positioning of the prosthetic heart valve, since he/she may, at first, decide to only expand one portion, for example the upper portion, in order to attach the attachment means to the native aortic valve and, in a second stage, make the lower portion expand in order to complete the positioning of the prosthetic heart valve.

The lower portion has a flared shape.

According to another aspect of the invention, the lower portion comprises a spongy casing suitable to reduce paravalvular leaks, or regurgitation. The casing has the advantageous function of bridging any differences in size between the prosthesis and the valve anatomy.

According to another aspect of the invention, the support structure is made of intertwined nitinol fibers.

This aspect advantageously provides the support structure with a filter function that allows to prevent the embolization of any debris that may detach during the manipulation of the native aortic valve.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic and partly sectioned view of a prosthetic heart valve according to the present invention in a compressed position and inserted into a catheter;
- fig. 2 is a three-dimensional schematic view of a prosthetic heart valve according to the present invention in an expanded position and extracted from the catheter;
- fig. 3 is a detail of a connector according to some embodiments of the present invention;
- fig. 4 is a schematic view of a prosthetic heart valve in accordance with one embodiment of the present invention in an expanded position;
- fig. 5 is a schematic view of a prosthetic heart valve in accordance with another embodiment of the present invention in an expanded position;
- fig. 6 is a detail of fig. 4 in accordance with one embodiment of the present invention;
- figs. from 7 to 12 are schematic and partly sectioned views of an operating sequence for replacing the native aortic valve A with the prosthetic heart valve of fig. 5;
- fig. 13 is a schematic view of a detail of fig. 12.

We must clarify that in the present description the phraseology and terminology used have the sole function of better illustrating the present invention with reference to the drawings and must not be in any way used to limit the scope of the invention itself, or the field of protection defined by the attached claims.

Furthermore, the people of skill in the art will recognize that certain sizes or characteristics in the drawings may have been enlarged, deformed, or shown in an unconventional or non-proportional way in order to provide a version of the present invention that is easier to understand.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS

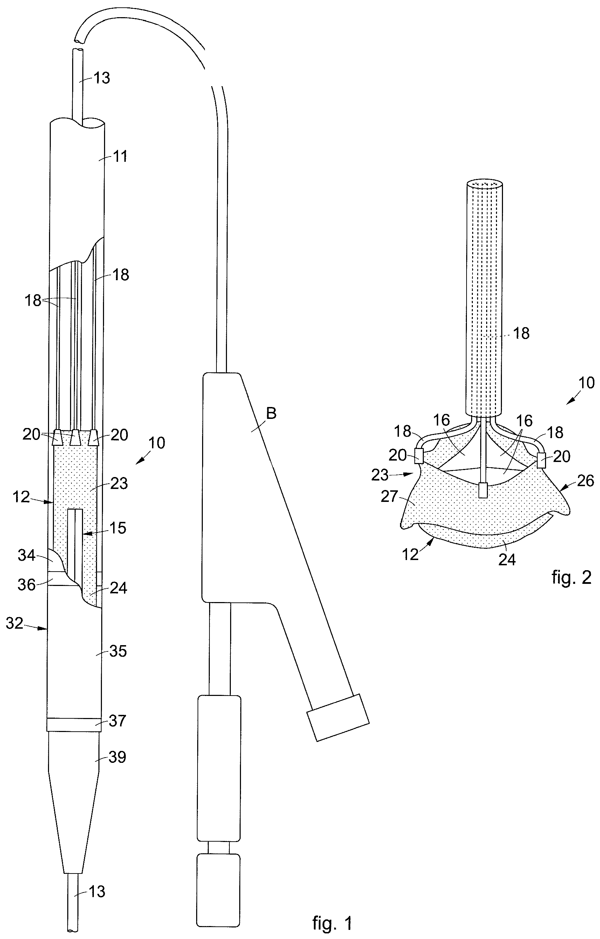

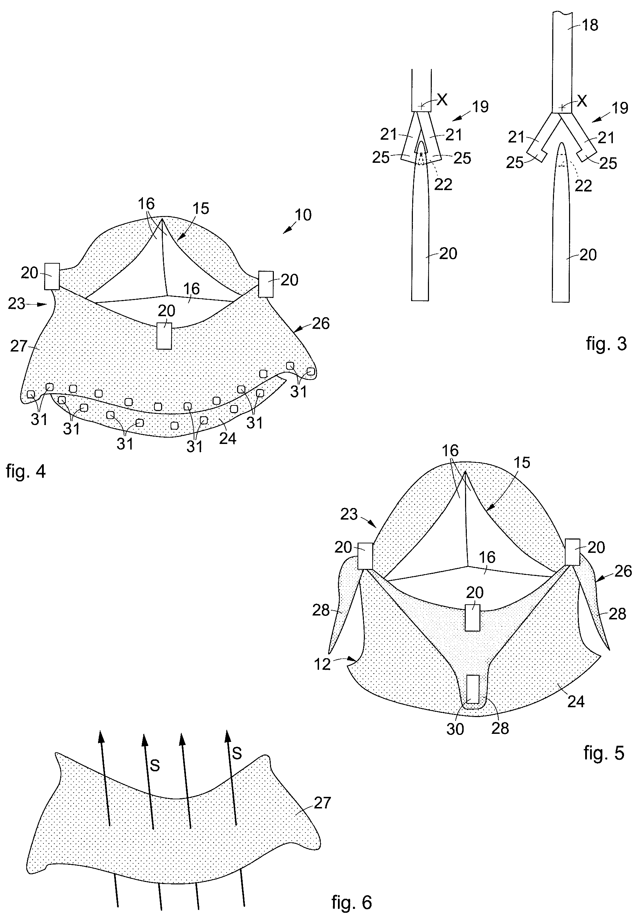

With reference to fig. 1, a prosthetic heart valve of the self-expandable type, for the percutaneous treatment of heart defects, in particular for the replacement of the native aortic valve A (figs. 6-11) of a heart, is indicated overall with reference number 10.

The prosthetic heart valve 10 can be inserted into a catheter 11 in order to be introduced into the body of a subject by means of a suitable insertion tool B of a known type.

The catheter 11 comprises a guide wire 13 which can be inserted therein and is suitable to make at least the prosthetic heart valve 10 slide.

The prosthetic heart valve 10 comprises a support structure 12 configured to self-expand in a substantially radial manner from a first compressed conformation, when inserted into the catheter 11, to a second expanded, or implant, conformation (fig. 2), when removed from the catheter 11.

The prosthetic heart valve 10 also comprises a valve prosthesis 15, configured to replace the native aortic valve A, disposed inside the support structure 12 and configured to assume a conformation that is coordinated with the conformation of the support structure 12. The valve prosthesis 15 is provided, in this specific case, with three cusps 16 made, preferably, with heart tissue deriving from pigs or suchlike.

The support structure 12 can be advantageously made of intertwined nitinol fiber, which is a shape-memory metal alloy, so that once compressed it is able to regain its original conformation, which is known a priori.

The support structure 12 is of the intra-annular type, that is, when the prosthetic heart valve 10 is implanted, the valve prosthesis 15 is located inside the native aortic valve A and it has a low profile, that is, its height does not extend beyond the native valve plane.

According to one aspect of the invention, the prosthetic heart valve 10 also comprises three connection cables 18 which can be inserted into the catheter 11; the connection cables 18 can be connected, at a first end, or proximal end, to the insertion tool B, and at another end to the support structure 12, in a removable manner, in order to allow the selective and reversible control, by means of the insertion tool B, of the conformation of the support structure 12.

Each connection cable 18 is terminally provided with a pincer mean 19 (fig. 3) configured to connect to the support structure 12 in a removable manner.

The prosthetic heart valve 10 also comprises three connectors 20, attached to the support structure 12 and configured to be grasped by a corresponding pincer mean 19.

In particular, each connector 20 is oblong in shape and has a first end attached to the support structure 12 and a second free end provided with a transverse through hole 22.

Each pincer mean 19 comprises two arms 21 hinged at the end of the respective connection cable 18 in order to rotate about an axis of rotation X, and each of the arms 21 is provided with a pin 25 shaped to be inserted in the through hole 22.

The rotation of the two arms 21, which substantially corresponds to the opening/closing movement of the pincer mean 19, can be selectively controlled by the insertion tool B, controlled by the operator. Each connection cable 18 also comprises the components, not shown here, suitable to control at least the opening/closing of the respective pincer mean 19.

The support structure 12 comprises an upper portion, or crown, 23 configured to be attached to the native aortic valve A, and a lower portion, or body, 24, which contains the valve prosthesis 15.

The lower portion 24 has a downward flared shape to further improve the attachment and stabilization of the support structure 12 at the level of the aortic valve ring of the native aortic valve A.

The lower portion 24 can comprise, for example, it can be wrapped by, a spongy casing, not shown here, suitable to reduce paravalvular leaks, or regurgitation.

The upper portion 23 is provided with attachment means 26 that have a first end associated with the support structure 12 and a second free end configured to be attached to the native aortic valve A, in particular in correspondence with its cusps.

In one embodiment, the attachment means 26 comprise a ring-type structure 27 (fig. 2) which extroflects from the upper portion 23.

In an alternative embodiment, the attachment means 26 comprise three elongated elements, or arms 28 (fig. 4), independent of each other, one for each cusp of the native aortic valve A.

Advantageously, each connector 20 can be associated with a corresponding elongated element 28 (fig. 5), or, in the case of the ring-type structure 27 (fig. 4), each of them is separated from the other by a predefined distance similar to the one that separates the connectors 20 in the representation of fig. 5.

In this way, it is possible to control the conformation of each elongated element 28 or of a portion of the ring-type structure 27 in an independent manner.

Each elongated element 28 comprises, in correspondence with the free end, a radiopaque probe 30 (only one is shown in fig. 5) which advantageously allows to provide the surgeon with a reference, when the radiopaque probe 30 is suitably detected, to correctly position the elongated elements 28 in correspondence with a respective cusp of the native aortic valve A.

Advantageously, the ring-type structure 27 does not require the presence of radiopaque probes, since its circular extension facilitates the association with the cusps of the native aortic valve A without the need for references.

In some embodiments, the prosthetic heart valve 10 also comprises a plurality of magnetic elements 31 (fig. 4), positioned in the lower portion 24 and, for example, in the ring-type structure 27, in particular in correspondence with its free end.

When the support structure 12 is in its expanded configuration, the magnetic elements 31 of the lower portion 24 and the magnetic elements 31 of the attachment means 26 are sufficiently close for a magnetic attraction force to form between them.

Consequently, the expanded conformation of the prosthetic heart valve 10 is more stable, thereby improving its anchorage to the native aortic valve A.

In one embodiment, the ring-type structure 27 is made of nitinol fibers intertwined to form a mesh, or grid, suitable to filter the debris released during implant operations and carried by a flow of blood S (fig 6).

The prosthetic heart valve 10 is inserted inside a capsule 32 (fig. 1) which is positioned at one end of the catheter 11, corresponding, when in use, to a distal position with respect to the medical operator.

The capsule 32 comprises a proximal portion 34 and a distal portion 35 connected to each other in a separable manner and sliding on the guide wire 13.

It is possible to separate the proximal portion 34 from the distal portion 35 by making one or the other slide independently.

The support structure 12 is inserted into the capsule 32 so that the upper portion 23 is contained in the proximal portion 34, while the lower portion 24 is contained in the distal portion 35.

In particular, between the proximal portion 34 and the distal portion 35 there is, for example associated with the distal portion 35, a proximal radiopaque probe 36 that has the function of indicating, when suitably detected, the boundary between the upper portion 23 and the lower portion 24 of the support structure 12.

The capsule 32 also comprises a distal radiopaque probe 37 associated with the distal portion 35 with the function of reference, when suitably detected, during the operation of release of the prosthetic heart valve 10 from the insertion tool B.

The catheter 11 terminates with a final element 39, as widely known in the state of the art.

With reference to figs. from 7 to 13, we will describe in sequence the operations for positioning the prosthetic heart valve 10 in correspondence with the native aortic valve A, in this specific case with a mode that is top-down with respect to the native aortic valve A, after its insertion by means of a catheter in proximity to the native valve A.

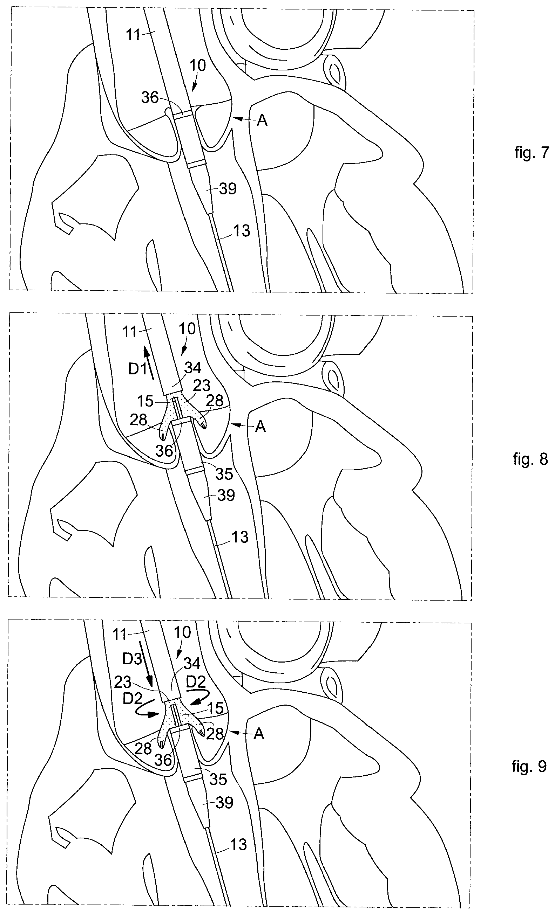

The actual positioning of the prosthetic heart valve 10 begins with a first step in which it is positioned in correspondence with the native aortic valve A (fig. 7) thanks to the help of the proximal radiopaque probe 36; subsequently, the upper portion 23 is extracted (fig. 8), so that the valve 10 begins to reacquire the expanded conformation, at least partly withdrawing the proximal portion 34 of the capsule 32 in direction D1.

With reference to fig. 9, in this step the medical operator, by acting on the insertion tool B, can impart a movement with direction D2, substantially of counterclockwise or clockwise rotation about an axis of longitudinal development of the catheter 11, to the support structure 12 through which the medical operator is able to orient the elongated elements 28, detecting the position of the radiopaque probes 34, in order to center each of the radiopaque probes 34 in correspondence with the respective cusp of the native aortic valve A. This step may not be provided in the event the upper portion 23 is provided with the ring-type structure 27.

The expansion of the upper portion 23 occurs in part passively, thanks to the shape-memory of the material with which it is made, while it exits from the corresponding portion of capsule 32, and in part actively, providing a movement with direction D3, that is, anterograde to the connection cables 18 which are coupled to the upper portion 23.

The connection of the upper portion 23 by means of the connection cables 18 allows to control the prosthetic heart valve 10 and to stabilize it even when the upper portion 23 is completely expanded inside the sinuses of Valsalva, and the possibility of withdrawing it again inside the capsule 32, if necessary.

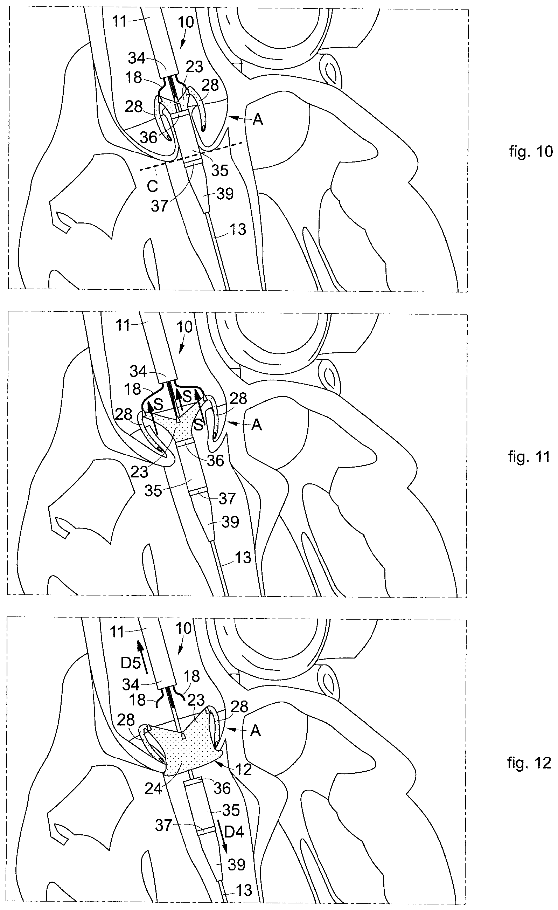

With reference to fig. 10, this shows the complete deployment of the upper portion 23 inside the sinuses of Valsalva in correspondence with the aortic cusps of the native aortic valve A.

At this point, it is provided to detect the correct positioning of the distal radiopaque probe 37, which has to be below the plane of the aortic ring C in the so-called "co-planar" angiographic projection.

With reference to fig. 11, this shows the partly expanded prosthetic heart valve 10, in particular with the upper portion 23 expanded and the lower portion 24 contained in the distal portion 35 of the capsule 32. The blood flow S that moves from the left ventricle toward the aorta, and which can possibly carry some debris released during implant operations, can be filtered by the upper portion 23 of the valve 10 when it is provided with the ring-type structure 27 according to the embodiment of fig. 6.

With reference to fig. 12, a second step is shown in which it is provided to extract the lower portion 24 so that the valve 10 begins to reacquire the expanded conformation, moving away the distal portion 35 of the capsule 32 by providing a movement with direction D4 opposite to the direction D1. Subsequently, it is provided to release the support structure 12 from the connection cables 18 and to withdraw the catheter 11, the capsule 32 and, finally, the guide wire 13, each with a movement in the direction D5.

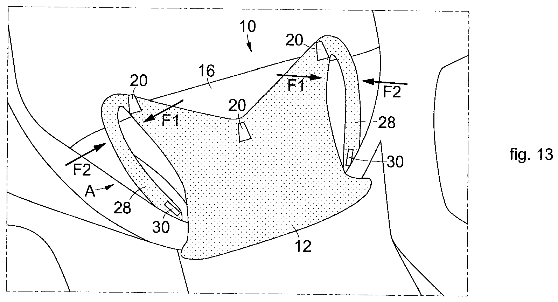

At this point, a radial force F1 (fig. 13) of expansion of the lower portion 24 against the upper portion 23, to which there is possibly added an attraction force F2 of the magnetic type due to the presence of the magnetic elements 31, not shown here for simplicity, guarantees the attachment of the casing 12 to the three aortic cusps of the native aortic valve A. If the need arises to recover the support structure 12, naturally before the third step, it is possible to act on the connection cables 18 with retrograde movement, in order to retract the upper portion 23 inside the capsule 32, subsequently close the proximal portion 34, retract the lower portion 24 inside the capsule 32 and, finally, make the support structure 12 advance inside the capsule 32 in order to position it in the starting position.

It is clear that modifications and/or additions of parts may be made to the prosthetic heart valve 10 as described heretofore, without departing from the field and scope of the present invention as defined by the claims. In the following claims, the sole purpose of the references in brackets is to facilitate reading and they must not be considered as restrictive factors with regard to the field of protection claimed in the specific claims.

## Claims

1. A system comprising a prosthetic heart valve and a catheter, the prosthetic heart valve (10), of the self-expandable type, for the percutaneous correction of a native aortic valve (A), wherein said prosthetic heart valve (10) is insertable into the catheter (11) to be introduced into a body by means of an insertion tool (B) which is associated with said catheter (11), said prosthetic heart valve (10) comprising:
a support structure (12) configured to self-expand in a substantially radial manner from a first compressed conformation when inserted into said catheter (11), to a second expanded, or implant, conformation, when removed from said catheter (11),
a valve prosthesis (15), configured to replace said native aortic valve (A), disposed inside said support structure (12), and connection means (18) which are insertable into said catheter (11) and which are connected, at a first end, or proximal end, to said insertion tool (B) and at a second opposite end, or distal end, to said support structure (12) in a removable manner, said connection means (18) being configured to allow the selective and reversible control, by means of said insertion tool (B), of the conformation of said support structure (12),
wherein said support structure (12) comprises an upper portion (23) provided with attachment means (26) which are configured to be attached to said native aortic valve (A) with which said connection means (18) are associated, and a lower portion (24) that contains said valve prosthesis (15),
wherein said attachment means (26) have a first end associated with the support structure (12) and a second free end configured to be attached to the cusps of the native aortic valve (A), said attachment means (26) comprising a ring-type structure (27) which extroflects from said upper portion (23) or a plurality of elongated arms (28), independent of each other, one for each cusp of the native aortic valve (A),
wherein said prosthetic heart valve (10) is inserted inside a capsule (32) which is positioned at one end of the catheter, the capsule having a proximal portion (34) and a distal portion (35) connected to each other in a separable manner,
wherein said upper portion (23) of the support structure (12) is contained in said proximal portion (34) and said lower portion (24) of the support structure (12) is contained in said distal portion (35), and
wherein said lower portion (24) has a flared shape to further improve the attachment and stabilization of the support structure (12) at the level of the aortic valve ring of the native aortic valve (A).

2. System as in claim 1, **characterized in that** said upper portion (23) comprises a plurality of connectors (20) configured to be associated with said connection means (18).

3. System as in claim 2, **characterized in that** said connection means (18) are provided with coupling means (19) configured to connect in a removable manner to each of said connectors (20).

4. System as in claim 3, **characterized in that** said coupling means (19) comprise two arms (21) hinged at the end of the respective connection means (18) in order to rotate about an axis of rotation (X) so as to be associated with said connectors (20), their rotation being selectively controllable by said insertion tool (B).

5. System as in any previous claim, **characterized in that** said lower portion (24) and said attachment means (26) comprise a plurality of magnetic elements (31) so that in said expanded conformation said plurality of magnetic elements (31) of said lower portion (24) and of said attachment means (26) are sufficiently close for a magnetic attraction force to form between them.

6. System as in any previous claim, **characterized in that** said lower portion (24) comprises a spongy casing suitable to reduce paravalvular leaks, or regurgitation.

7. System as in any previous claim, **characterized in that** said ring-type structure (27) is made of nitinol fibers intertwined to form a mesh suitable to filter debris released during implant operations.

## Patentansprüche

1. System umfassend eine Herzklappenprothese und einen Katheter, wobei die Herzklappenprothese (10), der selbstexpandierbaren Art, der perkutanen Korrektur einer nativen Aortenklappe (A) dient, worin die Herzklappenprothese (10) in den Katheter (11) einfügbar ist, damit sie in einen Körper mittels eines Einfügungswerkzeugs (B) eingeführt wird, das dem Katheter (11) zugeordnet ist, wobei die Herzklappenprothese (10) umfasst:
eine Stützstruktur (12), die dafür ausgelegt ist, sich selbst in einer im Wesentlichen radialen Weise von einer ersten komprimierten Gestaltung, wenn sie in den Katheter (11) eingefügt ist, zu einer zweiten expandierten, oder Implantats-, Gestaltung, wenn sie vom Katheter (11) entfernt ist, zu expandieren,
eine Klappenprothese (15), die dafür ausgelegt ist, die native Aortenklappe (A) zu ersetzen, die innerhalb der Stützstruktur (12) angeordnet ist, und
Verbindungsmittel (18), die in den Katheter (11) einfügbar sind und die, an einem ersten Ende, oder proximalen Ende, mit dem Einfügungswerkzeug (B) und an einem zweiten entgegengesetzten Ende, oder distalen Ende, mit der Stützstruktur (12) in einer abnehmbaren Weise verbunden sind, wobei die Verbindungsmittel (18) dafür ausgelegt sind, die selektive und reversible Steuerung, mittels des Einfügungswerkzeugs (B), der Gestaltung der Stützstruktur (12) zu ermöglichen,
worin die Stützstruktur (12) einen oberen Teil (23), der mit Anbringungsmitteln (26) versehen ist, die dafür ausgelegt sind, an der nativen Aortenklappe (A) angebracht zu werden, der die Verbindungsmittel (18) zugeordnet sind, und einen unteren Teil (24), der die Klappenprothese (15) enthält, umfasst,
worin die Anbringungsmittel (26) ein erstes Ende, das der Stützstruktur (12) zugeordnet ist, und ein zweites freies Ende, das dafür ausgelegt ist, an den Segeln der nativen Aortenklappe (A) angebracht zu werden, haben, wobei die Anbringungsmittel (26) eine ringförmige Struktur (27) umfassen, die sich vom oberen Teil (23) oder einer Vielzahl von länglichen Armen (28), unabhängig voneinander, einer für jedes Segel der nativen Aortenklappe (A), ausstülpt,
worin die Herzklappenprothese (10) in eine Kapsel (32) eingefügt ist, die an einem Ende des Katheters positioniert ist, wobei die Kapsel einen proximalen Teil (34) und einen distalen Teil (35) hat, die miteinander in einer trennbaren Weise verbunden sind,
worin der obere Teil (23) der Stützstruktur (12) in dem proximalen Teil (34) enthalten ist und der untere Teil (24) der Stützstruktur (12) in dem distalen Teil (35) enthalten ist,
und
worin der untere Teil (24) eine aufgeweitete Form hat, um die Anbringung und Stabilisierung der Stützstruktur (12) auf der Höhe des Aortenklappenringes der nativen Aortenklappe (A) weiter zu verbessern.

2. System nach Anspruch 1, **dadurch gekennzeichnet, dass** der obere Teil (23) eine Vielzahl von Verbindern (20) umfasst, die dafür ausgelegt sind, den Verbindungsmitteln (18) zugeordnet zu werden.

3. System nach Anspruch 2, **dadurch gekennzeichnet, dass** die Verbindungsmittel (18) mit Kopplungsmitteln (19) versehen sind, die dafür ausgelegt sind, sich in einer abnehmbaren Weise mit jedem der Verbinder (20) zu verbinden.

4. System nach Anspruch 3, **dadurch gekennzeichnet, dass** die Kopplungsmittel (19) zwei Arme (21) umfassen, die an dem Ende des jeweiligen Verbindungsmittels (18) angelenkt sind, um sich um eine Drehachse (X) zu drehen, so dass sie den Verbindern (20) zugeordnet werden, wobei ihre Drehung vom Einfügungswerkzeug (B) selektiv steuerbar ist.

5. System nach einem der vorhergehenden Ansprüche, **dadurch gekennzeichnet, dass** der untere Teil (24) und die Anbringungsmittel (26) eine Vielzahl von magnetischen Elementen (31) umfassen, so dass in der expandierten Gestaltung die Vielzahl von magnetischen Elementen (31) des unteren Teils (24) und der Anbringungsmittel (26) ausreichend nahe sind, damit sich eine magnetische Anziehungskraft zwischen ihnen bildet.

6. System nach einem der vorhergehenden Ansprüche, **dadurch gekennzeichnet, dass** der untere Teil (24) ein schwammiges Gehäuse umfasst, das dazu geeignet ist, paravalvuläre Lecks, oder Regurgitation, zu reduzieren.

7. System nach einem der vorhergehenden Ansprüche, **dadurch gekennzeichnet, dass** die ringförmige Struktur (27) aus Nitinol-Fasern hergestellt ist, die ineinander verflochten sind, um eine Masche zu bilden, die dazu geeignet ist, Abfälle, die während Implantationsoperationen freigegeben werden, zu filtern.

## Revendications

1. Système comprenant une valvule cardiaque prothétique et un cathéter, la valvule cardiaque prothétique (10), du type auto-expansible, pour la connexion percutanée d'une valvule aortique native (A), dans lequel ladite valvule cardiaque prothétique (10) peut être insérée dans le cathéter (11) pour être introduite dans un corps au moyen d'un outil d'insertion (B) qui est associé audit cathéter (11), ladite valvule cardiaque prothétique (10) comprenant :
une structure de support (12) configurée pour s'auto-expanser de manière sensiblement radiale à partir d'une première conformation comprimée lorsqu'elle est insérée dans ledit cathéter (11), à une seconde conformation expansée ou implantée, lorsqu'elle est retirée dudit cathéter (11),
une prothèse de valvule (15), configurée pour remplacer ladite valvule aortique native (A), disposée à l'intérieur de ladite structure de support (12), et
des moyens de connexion (18) qui peuvent être insérés dans ledit cathéter (11) et qui sont connectés, à une première extrémité, ou extrémité proximale, audit outil d'insertion (B) et à une seconde extrémité opposée, ou extrémité distale, à ladite structure de support (12) de manière amovible, lesdits moyens de connexion (18) étant configurés pour permettre la commande sélective et réversible, au moyen dudit outil d'insertion (B), de la conformation de ladite structure de support (12),
dans lequel ladite structure de support (12) comprend une partie supérieure (23) pourvue de moyens de fixation (26) qui sont configurés pour être fixés à ladite valvule aortique native (A) à laquelle lesdits moyens de connexion (18) sont associés, et une partie inférieure (24) qui contient ladite prothèse de valvule (15),
dans lequel lesdits moyens de fixation (26) ont une première extrémité associée à la structure de support (12) et une seconde extrémité libre configurée pour être fixée aux pointes de la valvule aortique native (A), lesdits moyens de fixation (26) comprenant une structure de type annulaire (27) qui s'extrafléchit à partir de ladite partie supérieure (23) ou d'une pluralité de bras allongés (28), indépendants les uns des autres, un pour chaque pointe de la valvule aortique native (A),
dans lequel ladite valvule cardiaque prothétique (10) est insérée à l'intérieur d'une capsule (32) qui est positionnée à une extrémité du cathéter, la capsule ayant une partie proximale (34) et une partie distale (35) reliées l'une à l'autre de manière séparable,
dans lequel ladite partie supérieure (23) de la structure de support (12) est contenue dans ladite partie proximale (34) et ladite partie inférieure (24) de la structure de support (12) est contenue dans ladite partie distale (35), et
dans lequel ladite partie inférieure (24) présente une forme évasée pour améliorer davantage la fixation et la stabilisation de la structure de support (12) au niveau de l'anneau de valvule aortique de la valvule aortique native (A).

2. Système selon la revendication 1, **caractérisé en ce que** ladite partie supérieure (23) comprend une pluralité de connecteurs (20) configurés pour être associés auxdits moyens de connexion (18).

3. Système selon la revendication 2, **caractérisé en ce que** lesdits moyens de connexion (18) sont pourvus de moyens de couplage (19) configurés pour se connecter de manière amovible à chacun desdits connecteurs (20).

4. Système selon la revendication 3, **caractérisé en ce que** lesdits moyens de couplage (19) comprennent deux bras (21) articulés à l'extrémité des moyens de connexion (18) respectifs afin de tourner autour d'un axe de rotation (X) de manière à être associés auxdits connecteurs (20), leur rotation pouvant être sélectivement commandée par ledit outil d'insertion (B).

5. Système selon l'une quelconque des revendications précédentes, **caractérisé en ce que** ladite partie inférieure (24) et lesdits moyens de fixation (26) comprennent une pluralité d'éléments magnétiques (31) de sorte que dans ladite conformation expansée, ladite pluralité d'éléments magnétiques (31) de ladite partie inférieure (24) et desdits moyens de fixation (26) sont suffisamment proches pour qu'une force d'attraction magnétique se forme entre eux.

6. Système selon l'une quelconque des revendications précédentes, **caractérisé en ce que** ladite partie inférieure (24) comprend un boîtier spongieux approprié pour réduire les fuites paravalvulaires ou la régurgitation.

7. Système selon l'une quelconque des revendications précédentes, **caractérisé en ce que** ladite structure de type annulaire (27) est constituée de fibres de nitinol entrelacées pour former une maille appropriée pour filtrer les débris libérés pendant les opérations d'implant.
